# EUROPEAN PATENT SPECIFICATION

(11) **EP 0 901 997 B1**
(45) Date of publication and mention of the grant of the patent: **31.07.2002**
(21) Application number: 98116570.7
(22) Date of filing: 02.09.1998
(51) Int. Cl.: C07C 29/145, B01J 31/24

(54) **Process for producing optically active alcohol compound**
Verfahren zur Herstellung optisch aktiver Alkohole
Procédé de préparation d'un alcool optiquement actif

(30) Priority: 05.09.1997 JP 24111997
(43) Date of publication of application: 17.03.1999
(73) Proprietor: Takasago International Corporation, Tokyo 108 (JP); T. Hasegawa Co., Ltd., Chuo-ku, Tokyo 103-8431 (JP)
(72) Inventor: Noyori, Ryoji, Nisshin-shi, Aichi (JP); Mikami, Koichi, Yokohama-shi, Kanagawa 231-0037 (JP); Ohkuma, Takeshi, Aichi-gun, Aichi (JP); Terada, Masahiro, Urawa-shi, Saitama (JP); Doucet, Henri, c/o Department of Chemistry, Furou-cho, Chikusa-ku, Nagoya-shi, Aichi (JP); Pham Thi Mai Trang, Catherine, c/o Dept. of Chem., Furou-cho, Chikusa-ku, Nagoya-shi, Aichi (JP); Korenaga, Toshinobu, Fujimi-shi, Saitama (JP); Ito, Masamichi, c/o T. Hasegawa Co., Ltd., Nakahara-ku, Kawasaki-shi, Kanagawa (JP); Sayou, Noboru, c/o Takasago International Corp., Hiratsuka-shi, Kanagawa (JP)
(74) Representative: HOFFMANN - EITLE

(56) References cited:
- EP-A- 0 718 265
- EP-A- 0 781 749

## Description

This invention relates to a process for the production of an optically active alcohol compound. More particularly, it relates to a novel and practically excellent process for the production of an optically active alcohol compound which is useful in various applications such as synthetic intermediates of perfumes, medicines, liquid crystal materials and the like.

### BACKGROUND OF THE INVENTION

Examples of currently known methods for the asymmetric synthesis of optically active alcohols include 1) a method in which an enzyme of baker's yeast or the like is used and 2) a method in which a carbonyl compound is subjected to asymmetric hydrogenation using a metal complex catalyst. Particularly in the latter method, many examples of asymmetric catalyst reaction have been reported. Examples of these reports include (1) a method described in Asymmetric Catalysis in Organic Synthesis, pp. 56 - 82 (1994), Ed., R. Noyori, in which asymmetric hydrogenation of a carbonyl compound having a functional group is carried out using an optically active ruthenium catalyst, (2) a method described in Chem. Rev., vol. 92, pp. 1051 - 1069 (1992), in which a hydrogen transfer type reduction is carried out using an asymmetric complex catalyst of ruthenium, rhodium or iridium, (3) a method described in Yukagaku, 822 - 831 (1980) and Advances in Catalysis, vol. 32, p. 215 (1983), Ed., Y. Izumi, in which asymmetric hydrogenation of tartaric acid is carried out using a modified nickel catalyst, (4) a method described in Asymmetic Syntheis, vol. 5, chap. 4 (1985), Ed., J.D. Morrison and J. Organomet, Chem., vol. 346, pp. 413 - 424 (1988), in which asymmetric hydrosilylation is carried out and (5) a method described in J. Chem. Soc., Perkin Trans., 1, 2039 - 2044 (1985) and J. Am. Chem. Soc., vol. 109, pp. 5551 - 5553 (1987), in which borane reduction is carried out in the presence of an asymmetric ligand.

However, though the method which uses an enzyme can produce alcohols having relatively high optical purity, it has disadvantages in that kinds of the reaction substrate are limited and absolute configurations of the obtained alcohols are also limited to certain specific types. Also, in the case of the method which uses an asymmetric hydrogenation catalyst of an transition metal, optically active alcohols can be produced with high selectivity from a keto acid or the like substrate which contains a functional group in its molecule, but this method has disadvantages in that it has a difficult point in terms of the reaction rate and it is not effective on a relatively simple carbonyl compound which does not have a functional group in its molecule.

In consequence, the present inventors have made an attempt to resolve these problems and found as the result that these problems can be resolved by carrying out asymmetric hydrogenation in the presence of an asymmetric hydrogenation catalyst, a base and an optically active nitrogen-containing compound (see JP-A-8-225466; the term "JP-A" as used herein means an "unexamined published Japanese patent application"), but this method was not necessarily satisfactory due to high cost of the optically active ligand to be used.

On the other hand, Mikami as one of the present inventors and others have reported in Nature, vol. 385, pp. 613- 615 (1997) that only one enantiomer of a racemic compound catalyst is activated when an optical activation agent is added to a catalyst of racemic body, and the catalyst becomes effective asymmetric catalyst as the result. This method, however, has such a limitation that it cannot be used in asymmetric hydrogenation reaction. Thus, great concern has been directed toward the development of a new synthetic method for use in the production of optically active alcohols, which uses a catalyst for popular use having high activity and low cost.

### SUMMARY OF THE INVENTION

In view of the above, the inventors of the present invention have conducted intensive studies with the aim of resolving these problems and found as a result of the efforts that, when two different complexes are formed in a reaction system by adding an optically active diamine compound as an activating agent to a racemic transition metal complex which is inexpensive in comparison with corresponding optically active substance or by adding a racemic diamine compound as an activating agent to an optically active transition metal complex, these two complexes show different reactivity to a carbonyl compound so that the carbonyl compound coordinates only to one of the complexes and its hydrogenation progresses, and an optically active alcohol can therefore be obtained with high optical purity and high yield. The present invention has been accomplished on the basis of this finding.

Accordingly, the present invention provide a process for producing an optically active alcohol compound represented by formula (II): 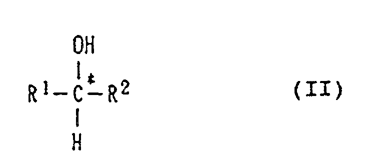 wherein R¹ represents an optionally substituted hydrocarbon group or an optionally substituted heterocyclic group, R² represents an optionally substituted hydrocarbon group or an optionally substituted heterocyclic group, or R² forms, together with R¹ and the adjacent carbon atom, an optionally substituted aliphatic ring, and * represents the location of an asymmetric carbon atom,
which comprises subjecting a carbonyl compound represented by formula (I): 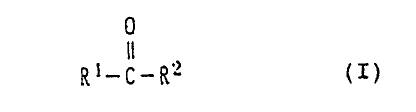 wherein R¹ and R² are as defined above,
to asymmetric hydrogenation in the presence of a transition metal complex containing a transition metal and a tertiary phosphine as a bidentate ligand, a base and a diamine, wherein the diamine is an optically active substance when the tertiary phosphine as a bidentate ligand is a racemic compound, or the diamine is a racemic compound when the tertiary phosphine as a bidentate ligand is an optically active substance.

### DETAILED DESCRIPTION OF THE INVENTION

In formula (I) which represents the carbonyl compound to be used as the starting material in the process of the present invention, the optionally substituted hydrocarbon or heterocyclic group represented by R¹ and R², is an aliphatic hydrocarbon group, a monocyclic or polycyclic aromatic hydrocarbon group, a monocyclic or polycyclic alicyclic hydrocarbon group or a monocyclic or polycyclic heterocyclic group, which may have a substituent group. Among these groups, examples of the aliphatic hydrocarbon group include saturated or unsaturated, straight- or branched-chain hydrocarbon groups, and more specifically alkyl groups such as methyl, ethyl, propyl, butyl, pentyl, hexyl, and heptyl groups and alkenyl groups such as vinyl and allyl groups. Illustrative examples of the monocyclic or polycyclic aromatic hydrocarbon group include phenyl, 2-methylphenyl, 2-ethylphenyl, 2-isopropylphenyl, 2-tert-butylphenyl, 2-methoxyphenyl, 2-chlorophenyl, 2-vinylphenyl, 3-methylphenyl, 3-ethylphenyl, 3-isopropylphenyl, 3-methoxyphenyl, 3-chlorophenyl, 3-vinylphenyl, 4-methylphenyl, 4-ethylphenyl, 4-isopropylphenyl, 4-tert-butylphenyl, 4-vinylphenyl, cumenyl, mesityl, xylyl, 1-naphthyl, 2-naphthyl, anthryl, phenanthryl and indenyl groups. Illustrative examples of the monocyclic or polycyclic alicyclic hydrocarbon group include cycloalkyl groups such as cyclopropyl, cyclobutyl, cyclopentyl and cyclohexyl groups. Illustrative examples of the monocyclic or polycyclic heterocyclic group include heterocyclic groups such as thienyl, furyl, pyranyl, xanthenyl, pyridyl, imidazolyl, indolyl, carbazolyl and phenanthrolyl groups. As their substituent groups, a halogen atom, a hydroxyl group, an alkoxyl group and the like can be exemplified. In addition, some groups formed by the mutual binding of the aforementioned hydrocarbon groups and heterocyclic groups, such as an aralkyl group, a ferrocenyl group and the like, can also be exemplified.

Examples of the aliphatic ring formed by R¹ and R² together with the adjacent carbon atom include 5- to 12-membered cyclic ketones such as cyclopentanone, cyclohexanone, cycloheptanone, cyclohexenone, cycloheptenone and the like. These cyclic ketones may be substituted with an alkyl group, an alkenyl group, an aromatic hydrocarbon group, a heterocyclic group, an alkoxyl group and the like.

Among these groups, an aliphatic hydrocarbon group, particularly an alkyl group, is desirable as R¹ and an aliphatic hydrocarbon group, an aromatic hydrocarbon group or a heterocyclic group is particularly desirable as R², and a case in which R¹ and R² together with the adjacent carbon atom form a cyclic ketone which may have a substituent group is also desirable.

Examples of the transition metal complex to be used in the present invention include a ruthenium phosphine complex represented by formula (III):

RuₘXₙLₚA_{q} (III)

wherein X represents a halogen atom, L represents a bidentate ligand compound of tertiary phosphine, A represents triethylamine (Et₃N) or dimethylformamide (DMF) and m, n, p and q are integers wherein m is 2, n is 4, p is 2 and q is 1 when A is Et₃N, or m is 1, n is 2, p is 1 and q is from 2 to 5 when A is DMF;
and a ruthenium phosphine compound represented by formula (IV):

[RuX(D)(L)]X (IV)

wherein X represents a halogen atom, L represents a bidentate ligand compound of tertiary phosphine, which is a racemic compound or an optically active substance, and D represents benzene, p-cymene, 1,3,5-trimethylbenzene or hexamethylbenzene.

Illustrative examples of L of the tertiary phosphine include a phosphine compound represented by formula (V): 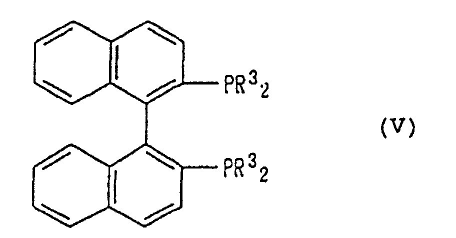 wherein R³ represents a phenyl group, a 4-methylphenyl group, a 3-methylphenyl group, a 3,5-dimethylphenyl group, a 4-methoxyphenyl group, a 3,5-dimethyl-4-methoxyphenyl group, a cyclohexyl group or a cyclopentyl group. Illustrative examples of compound other than that of formula (V) include a phosphine compound represented by formula (VI): 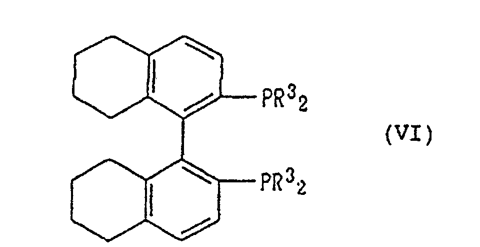 wherein R³ represents a phenyl group, a 4-methylphenyl group, a 4-methoxyphenyl group, a 3,5-dimethylphenyl group, a 4-methoxyphenyl group, a 3,5-dimethyl-4-methoxyphenyl group or a cyclohexyl group. Also, examples of compound other than that of formula (VI) include a phosphine compound represented by formula (VII): 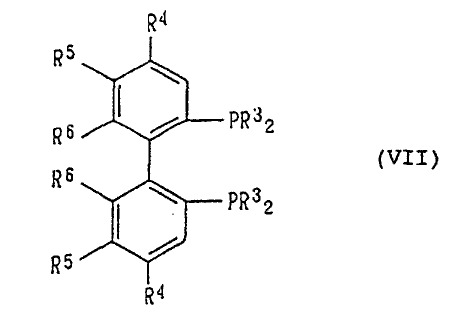 wherein R³ represents a phenyl group, a 4-methylphenyl group, a 4-methoxyphenyl group, a 3,5-dimethylphenyl group, a 4-methoxyphenyl group, a 3,5-dimethyl-4-methoxyphenyl group or a cyclohexyl group, R⁴ represents a hydrogen atom, a methyl group or a methoxy group, R⁵ represents a hydrogen atom, a methyl group, a methoxy group or a chlorine atom and R⁶ represents a methyl group, a methoxy group or a trifluoromethyl group.

Examples of useful optically active tertiary phosphine include (4,4',6,6'-tetramethyl-5,5'-dimethoxybiphenyl-2,2'-diyl)-bis(diphenylphosphine) and (4,4',6,6'-tetramethyl-5,5'-dimethoxybiphenyl-2,2'-diyl)-bis(di-p-methoxyphenylphosphine) disclosed in Chem. Pharm. Bull., 1991, vol. 39, p. 1085. Also useful are (4,4',6,6'-tetratrifluoromethylbiphenyl-2,2'-diyl)-bis(diphenylphosphine) and (4,6-ditrifluoromethyl-4',6'-dimethyl-5'-methoxybiphenyl-2,2'-diyl)-bis(diphenylphosphine) disclosed in Synlett, 1991, p. 827. Also useful is 2-dicyclohexyl-2'-diphenylphosphino-4,4',6,6'-tetramethyl-5,5'-dimethoxybiphenyl-2,2'-diyl)-bis(diphenylphosphine) disclosed in Tetrahedron: Asymmetry, 1992, vol. 3, p. 13. Also useful are (6,6'-dimethyl-2,2'-biphenylene)-bis(diphenylphosphine), (4,4',6,6'-tetramethyl-2,2'-biphenylene)-bis(diphenylphosphine), (3,3',6,6'-tetramethyl-2,2'-biphenylene)-bis(diphenylphosphine), (4,4'-difluoro-6,6'-dimethyl-2,2'-biphenylene)-bis(diphenylphosphine), (4,4'-bis(dimethylamino)-6,6'-dimethyl-2,2'-biphenylene)-bis(diphenylphosphine), (6,6'-dimethyl-2,2'-biphenylene)-bis(di-p-tolylphosphine), (6,6'-dimethyl-2,2'-biphenylene)-bis(di-o-tolylphosphine), (6,6'-dimethyl-2,2'-biphenylene)-bis(di-m-fluorophenylphosphine) and 1,11-bis(diphenylphosphino)-5,7-dihydrodibenzo[c,e]oxepin disclosed in JP-B-4-15796 (the term "JP-B" as used herein means an "examined Japanese patent publication"). Still useful are (6,6'-dimethoxybiphenyl-2,2'-diyl)-bis(diphenylphosphine), (5,5',6,6'-tetramethoxybiphenyl-2,2'-diyl)-bis(diphenylphosphine), (6,6'-dimethoxybiphenyl-2,2'-diyl)-bis(di-p-tolylphosphine) and (4,4',5,5',6,6'-tetrahexamethoxybiphenyl-2,2'-diyl)-bis(diphenylphosphine) disclosed in JP-A-3-5492.

In addition, examples of other compound than that of formula (VII) include a phosphine compound represented by formula (VIII): 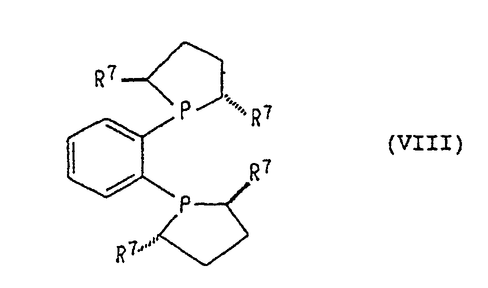 wherein R⁷ represents a methyl group, an ethyl group, a propyl group or an isopropyl group. Illustrative examples of this optically active tertiary phosphine include 1,2-bis(2,5-dimethylphosphorano)benzene, 1,2-bis(2,5-diethylphosphorano)benzene, 1,2-bis(2,5-dipropylphosphorano)benzene and 1,2-bis(2,5-diisopropylphosphorano)benzene disclosed in J. Am. Chem. Soc., 1993, vol. 115, p.10125. Also, examples of the compound other than that of formula (VIII) include CHIRAPHOS (2,3-bis-(diphenylphosphino)butane) (IX), PROPHOS (1,2-bis-(diphenylphosphino)propane) (X), NORPHOS (5,6-bis-(diphenylphosphino)-2-norbornane) (XI), DEGPHOS (1-substituted-3,4-bis-(diphenylphosphino)pyrrolidine) (XII), BDPP (2,4-bis-(diphenylphosphino)pentane) (XIII), DIOP (2,3-O-isopropylidene-2,3-dihydroxy-1,4-bis-(diphenylphosphino)butane) (XIV), DIPAMP (1,2-bis-[(o-methoxyphenyl)phenylphosphino]ethane) (XV), BPPHFOH (1-[1',2-bis-(diphenylphosphino)ferrocenyl]ethanol) (XVI), BPPM (1-tert-butoxycarbonyl-4-diphenylphosphino-2-diphenylphopsphinomethylpyrrolidine) (XVII) and the like.

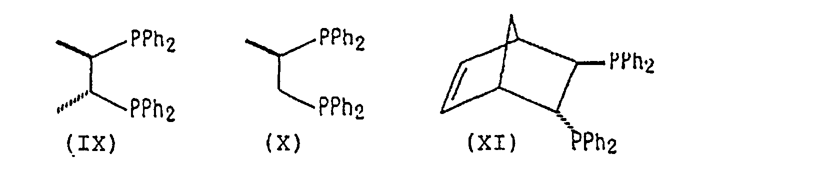

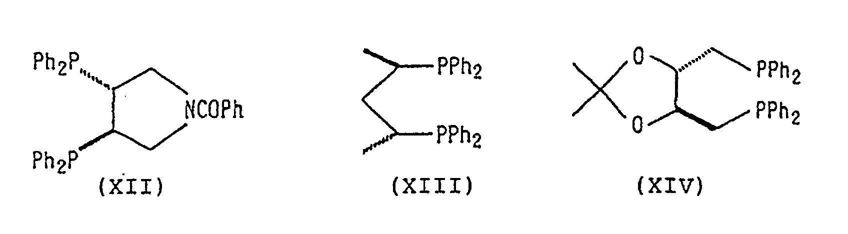

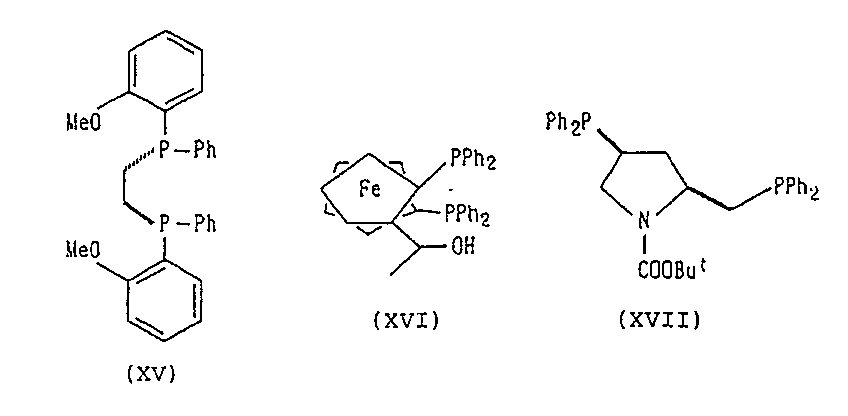

With regard to the base, an inorganic base or a quaternary ammonium salt can be exemplified, preferably an alkali metal compound or an alkaline earth metal compound and a quaternary ammonium salt, more preferably an alkali metal or alkaline earth metal hydroxide or a salt thereof and a quaternary ammonium salt. Examples of most preferred case is a metal salt represented by the following general formula (XVIII):

MY (XVIII)

wherein M represents an alkali metal or an alkaline earth metal and Y represents a hydroxy group, an alkoxy group, a mercapto group or a naphthyl group, or a quaternary ammonium salt.

Its illustrative examples include LiOH, LiOMe, LiOEt, LiOCH(CH₃)₂, LiOC(CH₃)₃, NaOH, NaOMe, NaOEt, NaOCH(CH₃)₂, NaOC(CH₃)₃, KOH, KOCH₃, KOCH(CH₃)₂, KOC(CH₃)₃, KC₁₀H₈ and the like. A quaternary ammonium salt can also be used.

The diamine compound of the present invention functions as an activator of the transition metal complex, and an optically active diamine compound is used when the transition metal complex is a racemic compound, or a racemic diamine compound is used when the transition metal complex is an optically active substance.

Examples of such diamine compounds include ethylenediamine compounds such as an optically active or racemic diamine compound represented by formula (XIX): 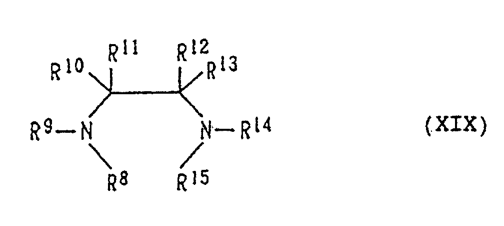 wherein R⁸, R⁹, R¹⁰, R¹¹, R¹², R¹³, R¹⁴ and R¹⁵ each represents a hydrogen atom or a saturated or unsaturated hydrocarbon group, an aryl group, a urethane group or a sulfonyl group, provided that R¹⁰, R¹¹, R¹² and R¹³ may be the same or different from one another wherein the carbon atom to which these substituent groups are linked becomes the asymmetric center and each represents a hydrogen atom, an alkyl group, a monocyclic or polycyclic aromatic hydrocarbon group, a saturated or unsaturated hydrocarbon group or a cyclic hydrocarbon group. Its illustrative examples include optically active or racemic ethylenediamine compounds such as optically active or racemic 1,2-diphenylethylenediamine, 1,2-cyclohexanediamine, 1,2-cycloheptanediamine, 2,3-dimethylbutanediamine, 1-methyl-2,2-diphenylethylenediamine, 1-isobutyl-2,2-diphenylethylenediamine, 1-isopropyl-2,2-diphenylethylenediamine, 1-methyl-2,2-di(p-methoxyphenyl)ethylenediamine, 1-isobutyl-2,2-di(p-methoxyphenyl)ethylenediamine, 1-isopropyl-2,2-di(p-methoxyphenyl)ethylenediamine, 1-benzyl-2,2-di(p-methoxyphenyl)ethylenediamine, 1-methyl-2,2-dinaphthylethylenediamine, 1-isobutyl-2,2-dinaphthylethylenediamine and 1-isopropyl-2,2-dinaphthylethylenediamine, as well as optically active diamine compounds in which one or two of the substituent groups R⁸ to R¹⁵ are a sulfonyl group or a urethane group. Also useful are optically active or racemic propanediamine, butanediamine and phenylenediamine derivatives. Particularly preferred diamine compound is diphenylethylenediamine.

According to the present invention, the three components, namely the transition metal complex to be used as a catalyst, the base and the diamine compound as an optical activation agent, are indispensable components for achieving high asymmetric yield through smooth progress of the asymmetric hydrogenation reaction, because an alcohol compound having high optical purity cannot be obtained with sufficient reaction activity when even one of these components becomes scarce.

The transition metal complex of the present invention may be used in an amount within the range of from 1/100 to 1/100,000, preferably within the range of from 1/500 to 1/10,000, as a molar ratio based on the carbonyl compound of formula (I) which is the reaction substrate, though the amount may vary depending on the reaction vessel, reaction system or economic situation. Also, the base may be used in an amount of preferably from 0.5 to 100 equivalents, more preferably from 2 to 40 equivalents, based on the transition metal complex. Also, the diamine compound may be used in an amount of preferably from 0.5 to 2.5 equivalents, more preferably from 1 to 2 equivalents, based on the transition metal complex.

According to the present invention, any optional solvent can be used with the proviso that it can solubilize the reaction materials and catalyst system. Its examples include aromatic hydrocarbon solvents such as toluene and xylene, aliphatic hydrocarbon solvents such as pentane and hexane, halogen-containing hydrocarbon solvents such as methylene chloride, ether solvents such as diethyl ether and tetrahydrofuran, alcohol solvents such as methanol, ethanol, 2-propanol, butanol and benzyl alcohol and hetero atom-containing organic solvents such as acetonitrile, dimethylformamide (DMF) and dimethyl sulfoxide (DMSO). Because the product is an alcohol, alcohol solvents are most preferred, and 2-propanol is particularly preferred. When a reaction solvent is hardly soluble in such a solvent, a mixed solvent may be used by selecting from the aforementioned solvents.

Amount of the solvent is judged based on the solubility of the reaction substrate and economic situation. In the case of 2-propanol, the reaction can be carried out at a substrate concentration of from a low concentration of 1% or less to close to a non-solvent state depending on each substrate, but it is desirable to use it in an amount of from 20 to 50% by weight.

According to the present invention, since the inventive catalyst system shows markedly high activity, 1 atm is sufficient as the hydrogen pressure which, however, may be preferably within the range of from 1 to 100 atm, more preferably within the range of from 3 to 50 atm, when economic situation is taken into consideration, and it is possible to maintain high activity even under 10 atm or less by taking economic situation of the whole process into consideration.

With regard to the reaction temperature, it is desirable to carry out the reaction at 0 to 40°C, but it can be carried out within the range of from -30 to 100°C. Also, the reaction time may preferably be within the range of from several minutes to 20 hours, more preferably from 1 to 20 hours, and particularly preferably from 1 to 10 hours, though it may vary depending on reaction conditions such as the substrate concentration, temperature, pressure and the like.

The reaction of the present invention can be carried out by either a batch system or a continuous system.

### EXAMPLES

Examples of the present invention are given below by way of illustration and not by way of limitation.

In this connection, physical property data in the examples were measured by the following measuring apparatuses and conditions.

### 1) Yield and optical purity;

### Gas chromatography (Examples 1 and 3)

- Apparatus:: HP-6890 (manufactured by Hewlett-Packard, Ltd.)
- Column:: Chirasil-DEX CB (0.32 mm x 25 m) (manufactured by Chrompack Co.)
- Temperature:: 90°C

### High performance liquid chromatography (Inventive Examples 5 and 6)

- Apparatus:: LC-6A (manufactured by Shimadzu Corp.)
- Column:: CHIRALPAK AS (manufactured by Daicel Chemical Industries, Ltd.)
- Solvent:: Example 3, hexane:2-propanol (100:1 by volume)
Example 5, hexane:2-propanol (98:2 by volume)
- Flow rate:: 1 ml/min.
- Detector:: Ultraviolet absorption photometer SPD-6A (254 nm) (manufactured by Shimadzu Corp.)

### 2) Proton nuclear magnetic resonance spectrum (¹H-NMR);

### <A> (Examples 1 and 3)

- Apparatus:: LAMBDA-500 (500 MHz) (manufactured by JEOL Ltd.)

### <B> (Examples 5 and 6)

- Apparatus:: GEMINI-300 (300 MHz) (manufactured by Varian Co.)

### 3) Angle of rotation;

- Apparatus:: DIP-370 (manufactured by JASCO Corp.)

### EXAMPLE 1

### Synthesis of (S)-2,4,4-trimethyl-2-cyclohexenol

A 200 ml glass autoclave was charged with RuCl₂[(R,S)-tolbinap](dmf)ₙ (9.4 mg, 0.01 mmol) and (S,S)-1,2-diphenylethylenediamine (2.1 mg, 0.01 mmol) and the atmosphere in the autoclave was replaced with argon gas, wherein "tolbinap" means "2,2'-bis(di-p-tolylphosphino)-1,1'-binaphthyl" and "dmf" means "dimethylformamide".

Into this were introduced 2-propanol (3 ml), toluene (1 ml) and 0.5 M KOH solution in 2-propanol (40 ml, 0.02 mol) in a stream of argon. The mixture was degassed repeatedly and was then sonicated for 15 minutes to obtain homogeneous solution. In a stream of argon, into this solution was introduced 2-propanol (4 ml) solution of 2,4,4-trimethyl-2-cyclohexenone (691 mg, 5.0 mmol) which has been subjected to degassing-argon replacement in advance. After cooling the autoclave in ice water, the reaction was started by forcing hydrogen under 8 atmospheric pressure, and the reaction solution was stirred for 6 hours at 0°C and then returned to ordinary temperature, subsequently replacing the atmosphere in the autoclave with argon. After concentration of the reaction solution, the thus obtained crude product was purified by a silica gel column chromatography (eluent, ether:n-hexane = 1:4 by volume) to obtain (S)-2,4,4-trimethyl-2-cyclohexenol (684 mg).
Yield: 98%
Optical purity: 95% e.e.
Angle of rotation: [α]_{D}²⁴ = -88.3°
¹H-NMR (CDCl₃) δ: 0.93 (s, 3H), 0.99 (3, 3H), 1.3 - 2.0 (m, 5H), 1.7 - 1.8 (m, 3H), 3.93 (br q, 1 H, J = 5.1 Hz), 5.24 (br s, 1 H).

### EXAMPLE 2

The reaction was carried out in the same manner as described in Example 1, except that RuCl₂[(R)-tolbinap](dmf)ₙ was used in stead of RuCl₂[(R,S)-tolbinap](dmf)ₙ and (±)-diphenylethylenediamine was used in stead of (S,S)-1,2-diphenylethylenediamine, thereby obtaining (S)-2,4,4-trimethyl-2-cyclohexenol as the formed product.
Yield: 99%
Optical purity: 90% e.e.

### COMPARATIVE EXAMPLE 1

The reaction was carried out in the same manner as described in Example 1, except that RuCl₂[(R)-tolbinap](dmf)ₙ (JP-A-8-225466) was used in stead of RuCl₂[(R,S)-tolbinap](dmf)ₙ, thereby obtaining (S)-2,4,4-trimethyl-2-cyclohexenol.
Yield: 98%
Optical purity: 96% e.e.

As is evident from the above results, it was able to obtain a product using an inexpensive racemic complex at the same level as the product produced using an expensive optically active complex.

### EXAMPLE 3

### Synthesis of (R)-1-(o-methylphenyl)ethanol

A 200 ml glass autoclave was charged with RuCl₂[(R,S)-tolbinap](dmf)ₙ (9.4 mg, 0.01 mmol) and (S,S)-1,2-diphenylethylenediamine (2.1 mg, 0.01 mmol) and the atmosphere in the autoclave was replaced with argon gas. Into this were introduced 2-propanol (3 ml), toluene (1 ml) and 1.0 M KOC(CH₃)₃ solution in tert-butyl alcohol (20 ml, 0.02 mol) in a stream of argon.

The mixture was degassed repeatedly and was then sonicated for 15 minutes to obtain homogeneous solution. In a stream of argon, into this solution was introduced 2-propanol (4 ml) solution of o-methylacetophenone (671 mg, 5.0 mmol) which has been subjected to degassing-argon replacement in advance.

After cooling the reaction autoclave in ice water, the reaction was started by forcing hydrogen under 4 atmospheric pressure, and the reaction solution was stirred for 10 hours at 0°C and then returned to ordinary temperature, subsequently replacing the atmosphere in the autoclave with argon.

By gas chromatography and NMR measurements of the reaction solution, (R)-1-(o-methylphenyl)ethanol was identified as the product and its yield was confirmed to be 99% or more.
Optical purity: 90% e.e.

### EXAMPLE 4

The reaction of Example 3 was repeated, except that RuCl₂[(R)-tolbinap](dmf)ₙ was used in stead of RuCl₂[(R,S)-tolbinap](dmf)ₙ and (±)-diphenylethylenediamine was used in stead of (S,S)-1,2-diphenylethylenediamine, thereby obtaining (S)-1-(o-methylphenyl)ethanol as the formed product.
Yield: 99%
Optical purity: 85% e.e.

### EXAMPLE 5

### Synthesis of (R)-1-(1-naphthyl)ethanol

The reaction of Example 1 was repeated, except that 1-acetonaphthone was used as the reaction substrate.

By high performance liquid chromatography and NMR measurements of the reaction solution, (R)-1-(1-naphthyl)ethanol was identified as the product and its yield was confirmed to be 99% or more.
Optical purity: 73% e.e.
¹H-NMR (CDCl₃) δ: 1.59 (d, 3 H, J = 6.6 Hz), 1.90 (d, 1 H, J = 3.6 Hz), 5.59 (dq, 1 H, J = 3.6, 6.6 Hz), 7.37 - 7.51 (m, 3 H), 7.60 (d, 1 H, J = 6.6 Hz), 7.70 (d, 1 H, J = 8.1 Hz), 7.78 - 7.81 (m, 1 H), 8.02 - 8.05 (m, 1 H).

### EXAMPLE 6

The reaction of Example 5 was repeated, except that RuCl₂[(S)-tolbinap](dmf)ₙ was used in stead of RuCl₂[(R,S)-tolbinap](dmf)ₙ and (±)-diphenylethylenediamine was used in stead of (S,S)-1,2-diphenylethylenediamine, thereby obtaining (R)-1-(1-naphthyl)ethanol.
Yield: 99%
Optical purity: 84% e.e.

### COMPARATIVE EXAMPLE 2

The reaction of Example 5 was repeated, except that RuCl₂[(S)-tolbinap](dmf)ₙ was used in stead of RuCl₂[(R,S)-tolbinap](dmf)ₙ, thereby obtaining (R)-1-(1-naphthyl)ethanol.
Yield: 99%
Optical purity: 98% e.e.

### EXAMPLE 7

### Synthesis of (R)-1-(9-anthryl)ethanol

The reaction of Example 1 was repeated, except that 9-acetylanthracene was used as the reaction substrate, thereby obtaining (R)-1-(9-anthryl)ethanol as the formed product.
Yield: 99%
Optical purity: 80% e.e.
¹H-NMR (CDCl₃) δ: 1.94 (d, 3 H, J = 6.6 Hz), 2.21 (d, 1 H, J = 2.1 Hz), 6.50 (dq, 1 H, J = 2.1, 6.6 Hz), 7.43 - 7.53 (m, 4 H), 8.00 - 8.03 (m, 2 H), 8.41 (s, 1 H), 8.69 (d, 2 H, J = 9.3 Hz).

### COMPARATIVE EXAMPLE 3

The reaction of Example 7 was repeated, except that RuCl₂[(S)-tolbinap](dmf)ₙ was used in stead of RuCl₂[(R,S)-tolbinap](dmf)ₙ, thereby obtaining (R)-1-(9-anthryl)ethanol.
Yield: 99%
Optical purity: 82% e.e.

According to the present invention, various optically active alcohols can be obtained with high purity and high yield and at low cost by an industrially advantageous method.

## Claims

1. A process for producing an optically active alcohol compound represented by formula (II): 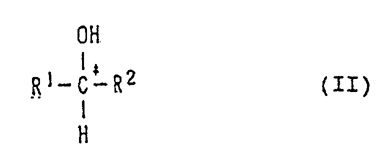 wherein R¹ represents an optionally substituted hydrocarbon group or an optionally substituted heterocyclic group, R² represents an optionally substituted hydrocarbon group or an optionally substituted heterocyclic group, or R² forms, together with R¹ and the adjacent carbon atom, an optionally substituted aliphatic ring, and * represents the location of an asymmetric carbon atom,
which comprises subjecting a carbonyl compound represented by formula (I): 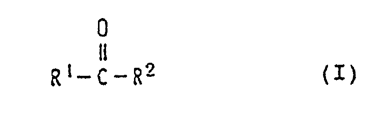 wherein R¹ and R² are as defined above,
to asymmetric hydrogenation in the presence of a transition metal complex containing a transition metal and a tertiary phosphine as a bidentate ligand, a base and a diamine wherein said diamine is an optically active substance when said tertiary phosphine as a bidentate ligand is a racemic compound, or said diamine is a racemic compound when said tertiary phosphine as a bidentate ligand is an optically active substance.

2. The process for producing an optically active alcohol compound according to claim 1, wherein said transition metal complex is a ruthenium phosphine complex represented by formula (III):
RuₘXₙLₚA_{q} (III)
wherein X represents a halogen atom, L represents a bidentate ligand compound of tertiary phosphine, A represents triethylamine (Et₃N) or dimethylformamide (DMF) and m, n, p and q are integers wherein m is 2, n is 4, p is 2 and q is 1 when A is Et₃N, or m is 1, n is 2, p is 1 and q is from 2 to 5 when A is DMF;
or a ruthenium phosphine compound represented by formula (IV):
[RuX(D)(L)]X (IV)
wherein X represents a halogen atom, L represents a bidentate ligand compound of tertiary phosphine, which is a racemic compound or an optically active substance, and D represents benzene, p-cymene, 1,3,5-trimethylbenzene or hexamethylbenzene.

3. The process for producing an optically active alcohol compound according to claim 1, wherein said diamine is an ethylenediamine derivative.

4. The process for producing an optically active alcohol compound according to claim 1, wherein said diamine is diphenylethylenediamine.

5. The process for producing an optically active alcohol compound according to claim 1, wherein said base is an alkali metal compound or an alkaline earth metal compound.

6. The process for producing an optically active alcohol compound according to claim 1, wherein said transition metal complex is used in an amount within the range of from 1/100 to 1/100,000 as a molar ratio based on the carbonyl compound of formula (I).

7. The process for producing an optically active alcohol compound according to claim 1, wherein said transition metal complex is used in an amount within the range of from 1/500 to 1/10,000 as a molar ratio based on the carbonyl compound of formula (I).

8. The process for producing an optically active alcohol compound according to claim 1, wherein said base is used in an amount of from 0.5 to 100 equivalents based on the transition metal complex.

9. The process for producing an optically active alcohol compound according to claim 1, wherein said base is used in an amount of from 2 to 40 equivalents based on the transition metal complex.

10. The process for producing an optically active alcohol compound according to claim 1, wherein said diamine is used in an amount of from 0.5 to 2.5 equivalents based on the transition metal complex.

11. The process for producing an optically active alcohol compound according to claim 1, wherein said diamine is used in an amount of from 1 to 2 equivalents based on the transition metal complex.

## Patentansprüche

1. Verfahren zur Erzeugung einer optisch aktiven Alkoholverbindung, dargestellt durch die Formel (II) : 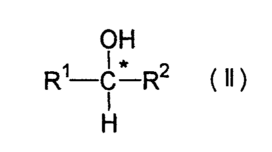 worin R¹ eine gegebenenfalls substituierte Kohlenwasserstoffgruppe oder eine gegebenenfalls substituierte heterocyclische Gruppe bedeutet, R² bedeutet eine gegebenenfalls substituierte Kohlenwasserstoffgruppe oder eine gegebenenfalls substituierte heterocyclische Gruppe oder R² bildet zusammen mit R¹ und dem benachbarten Kohlenstoffatom einen gegebenenfalls substituierten aliphatischen Ring und * bedeutet die Stellung eines asymmetrischen Kohlenstoffatoms,
umfassend das Unterwerfen einer Carbonylverbindung, dargestellt durch die Formel (I): 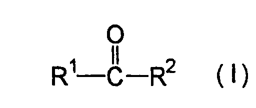 worin R¹ und R² wie oben definiert sind,
einer asymmetrischen Hydrierung in Gegenwart eines Übergangsmetallkomplexes, enthaltend ein Übergangsmetall und ein tertiäres Phosphin als zweizähnigen Liganden, einer Base und einem Diamin, wobei das Diamin eine optisch aktive Substanz ist, wenn das tertiäre Phosphin als zweizähniger Ligand eine racemische Verbindung ist, oder das Diamin ist eine racemische Verbindung, wenn das tertiäre Phosphin als zweizähniger Ligand eine optisch aktive Substanz ist.

2. Verfahren zur Erzeugung einer optisch aktiven Alkoholverbindung gemäss Anspruch 1, wobei der Übergangsmetallkomplex ein Ruthenium-Phosphin-Komplex ist, dargestellt durch Formel (III):
RuₘXₙLₚA_{q} (III)
worin X ein Halogenatom bedeutet, L bedeutet eine zweizähnige Ligandenverbindung eines tertiären Phosphins, A bedeutet Triethylamin (Et₃N) oder Dimethylformamid (DMF) und m, n, p und q sind ganze Zahlen, worin m 2 ist, n ist 4, p ist 2 und q ist 1, wenn A Et₃N ist, oder m ist 1, n ist 2, p ist 1 und q ist 2 bis 5, wenn A DMF ist;
oder eine Rutheniumphosphinverbindung, dargestellt durch Formel (IV) :
[RuX(D)(L)]X (IV)
worin X ein Halogenatom bedeutet, L bedeutet eine zweizähnige Ligandenverbindung eines tertiären Phosphins, die eine racemische Verbindung oder eine optisch aktive Substanz ist, und D bedeutet Benzol, p-Cymol, 1,3,5-Trimethylbenzol oder Hexamethylbenzol.

3. Verfahren zur Erzeugung einer optisch aktiven Alkoholverbindung gemäss Anspruch 1, wobei das Diamin ein Ethylendiaminderivat ist.

4. Verfahren zur Erzeugung einer optisch aktiven Alkoholverbindung gemäss Anspruch 1, wobei das Diamin Diphenylethylendiamin ist.

5. Verfahren zur Erzeugung einer optisch aktiven Alkoholverbindung gemäss Anspruch 1, worin die Base eine Alkalimetallverbindung oder eine Erdalkalimetallverbindung ist.

6. Verfahren zur Erzeugung einer optisch aktiven Alkoholverbindung gemäss Anspruch 1, wobei der Übergangsmetallkomplex in einer Menge im Bereich von 1:100 bis 1:100.000 als molares Verhältnis, basierend auf der Carbonylverbindung der Formel (I), verwendet wird.

7. Verfahren zur Erzeugung einer optisch aktiven Alkoholverbindung gemäss Anspruch 1, wobei der Übergangsmetallkomplex in einer Menge im Bereich von 1:500 bis 1:10.000 als molares Verhältnis, basierend auf der Carbonylverbindung der Formel (I), verwendet wird.

8. Verfahren zur Erzeugung einer optisch aktiven Alkoholverbindung gemäss Anspruch 1, wobei die Base in einer Menge von 0,5 bis 100 Äquivalenten, basierend auf dem Übergangsmetallkomplex, verwendet wird.

9. Verfahren zur Erzeugung einer optisch aktiven Alkoholverbindung gemäss Anspruch 1, wobei die Base in einer Menge von 2 bis 40 Äquivalenten, basierend auf dem Übergangsmetallkomplex, verwendet wird.

10. Verfahren zur Erzeugung einer optisch aktiven Alkoholverbindung gemäss Anspruch 1, wobei das Diamin in einer Menge von 0,5 bis 2,5 Äquivalenten, basierend auf dem Übergangsmetallkomplex, verwendet wird.

11. Verfahren zur Erzeugung einer optisch aktiven Alkoholverbindung gemäss Anspruch 1, wobei das Diamin in einer Menge von 1 bis 2 Äquivalenten, basierend auf dem Übergangsmetallkomplex, verwendet wird.

## Revendications

1. Procédé de préparation d'un composé alcool actif optiquement représenté par la formule (II): 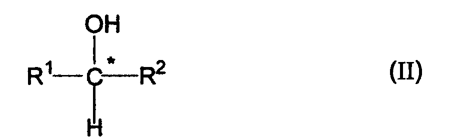 dans laquelle R¹ représente un groupe hydrocarboné substitué optiquement ou un groupe hétérocyclique éventuellement substitué, R² représente un groupe hydrocarboné substitué optiquement ou un groupe hétérocyclique éventuellement substitué, ou R² forme, avec R¹ et l'atome de carbone adjacent, un cycle aliphatique éventuellement substitué, et * représente la position d'un atome de carbone asymétrique,
qui comprend l'étape consistant à soumettre un composé carbonyle représenté par la formule (I) : 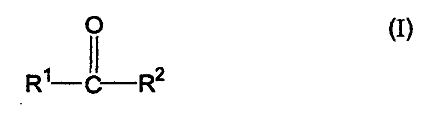 dans laquelle R¹ et R² sont tels que définis ci-dessus,
à une hydrogénation asymétrique en présence d'un complexe de métal de transition contenant un métal de transition et une phosphine tertiaire en tant que ligand bidentate, une base et une diamine, dans lequel ladite diamine est une substance active optiquement quand ladite phosphine tertiaire en tant que ligand bidentate est un composé racémique, ou ladite diamine est un composé racémique quand ladite phosphine tertiaire en tant que ligand bidentate est une substance active optiquement.

2. Procédé de préparation d'un composé alcool actif optiquement selon la revendication 1, dans lequel ledit complexe de métal de transition est un complexe de phosphine ruthénium représenté par la formule (III) :
RuₘXₙLₚA_{q} (III)
dans laquelle X représente un atome d'halogène, L représente un composé ligand bidentate de phosphine tertiaire, A représente la triéthylamine (Et₃N) ou le diméthylformamide (DMF) et m, n, p et q sont des nombres entiers où m vaut 2, n vaut 4, p vaut 2 et q vaut 1 quand A est la Et₃N, ou m vaut 1, n vaut 2, p vaut 1 et q vaut 2 à 5 quand A est le DMF;
ou un composé phosphine ruthénium représenté par la formule (IV) :
[RuX(D)(L)]X (IV)
dans laquelle X représente un atome d'halogène, L représente un composé ligand bidentate de phosphine tertiaire, qui est un composé racémique ou une substance active optiquement, et D représente le benzène, le p-cumène, le 1,3,5-triméthylbenzène ou l'hexaméthylbenzène.

3. Procédé de préparation d'un composé alcool actif optiquement selon la revendication 1, dans lequel ladite diamine est un dérivé d'éthylènediamine.

4. Procédé de préparation d'un composé alcool actif optiquement selon la revendication 1, dans lequel ladite diamine est la diphényléthylènediamine.

5. Procédé de préparation d'un composé alcool actif optiquement selon la revendication 1, dans lequel ladite base est un composé de métal alcalin ou un composé de métal alcalino-terreux.

6. Procédé de préparation d'un composé alcool actif optiquement selon la revendication 1, dans lequel ledit complexe de métal de transition est utilisé en une quantité dans la plage de 1/100 à 1/100 000 comme rapport molaire rapporté au composé carbonyle de formule (I).

7. Procédé de préparation d'un composé alcool actif optiquement selon la revendication 1, dans lequel ledit complexe de métal de transition est utilisé en une quantité dans la plage de 1/500 à 1/10 000 comme rapport molaire rapporté au composé carbonyle de formule (I).

8. Procédé de préparation d'un composé alcool actif optiquement selon la revendication 1, dans lequel ladite base est utilisée en une quantité de 0,5 à 100 équivalents rapporté au complexe de métal de transition.

9. Procédé de préparation d'un composé alcool actif optiquement selon la revendication 1, dans lequel ladite base est utilisée en une quantité de 2 à 40 équivalents rapporté au complexe de métal de transition.

10. Procédé de préparation d'un composé alcool actif optiquement selon la revendication 1, dans lequel ladite diamine est utilisée en une quantité de 0,5 à 2,5 équivalents rapporté au complexe de métal de transition.

11. Procédé de préparation d'un composé alcool actif optiquement selon la revendication 1, dans lequel ladite diamine est utilisée en une quantité de 1 à 2 équivalents rapporté au complexe de métal de transition.
